(19) Europäisches Patentamt
European Patent Office
Office européen des brevets

(11) Numéro de publication: **0 389 386 A1**

(12) **DEMANDE DE BREVET EUROPEEN**

(21) Numéro de dépôt: **90420107.6**

(22) Date de dépôt: **27.02.90**

(51) Int. Cl.5: **C08G 77/445**

(30) Priorité: **02.03.89 FR 8902995**

(43) Date de publication de la demande:
**26.09.90 Bulletin 90/39**

(84) Etats contractants désignés:
**AT BE CH DE DK ES FR GB GR IT LI LU NL SE**

(71) Demandeur: **RHONE-POULENC CHIMIE**
**25, quai Paul Doumer**
**F-92408 Courbevoie Cédex(FR)**

(72) Inventeur: **Helary, Gérard**
**7, Résidence des Epagnes**
**F-95640 Santeuil(FR)**
Inventeur: **Jorda, Rafael**
**30, rue Claude Jusseaud**
**F-69119 Sainte-Foy-Les-Lyon(FR)**
Inventeur: **Porte, Hugues**
**6, Chemin de Crépieux**
**F-69300 Caluire(FR)**
Inventeur: **Prud'Homme, Christian**
**19, rue du Commandant Faurax**
**F-69006 Lyon(FR)**
Inventeur: **Sauvet, Georges**
**5, rue Charles Renouvier**
**F-75020 Paris(FR)**
Inventeur: **Torres, Ghislaine**
**104, rue Ney**
**F-69006 Lyon(FR)**

(74) Mandataire: **Seugnet, Jean Louis et al**
**RHONE-POULENC CHIMIE Service Brevets**
**Chimie Centre de Recherches des Carrières**
**B.P. 62**
**F-69192 Saint-Fons Cédex(FR)**

(54) **Copolymère à blocs polyester-silicone dégradable par hydrolyse.**

(57) La présente invention concerne un copolymère à bloc diorganopolysiloxane-polyester dégradable par hydrolyse présentant de 1 à 99 % en poids de segment diorganopolysiloxane.

Les copolymères selon l'invention sont généralement thermoplastiques et dégradables par hydrolyse et peuvent être utilisés pour la fabrication de matrices érodables contenant une matière active médicament, produit phytosanitaire, etc .... en vue de sa libération contrôlée.

EP 0 389 386 A1

# COPOLYMERE A BLOCS POLYESTER-SILICONE DEGRADABLE PAR HYDROLYSE

La présente invention concerne un copolymère à blocs polyester-silicone, son procédé de préparation et son utilisation comme matrice ou comme pellicule d'une capsule contenant un principe actif en vue de la libération contrôlée du principe actif par simple érosion par hydrolyse de cette matrice ou capsule et/ou par diffusion du principe actif à travers la matrice ou capsule avant ou durant son érosion.

Dans ce type de système à libération contrôlée, la libération du principe actif qui est une substance minérale, organique ou végétale, est fonction de la nature du principe actif, de la matrice et du caractère érodable de la matrice qui déterminent le profil de libération du principe actif.

Les polymères dégradables par hydrolyse en particulier biodégradables déjà décrits dans la littérature sont principalement des polyéthylènes cyanoesters, des polyamides, des polyuréthannes, des polyacétates, des polylactones, des polyanhydrides, des polyorthoesters et des polyesters.

Les polymères silicones sont utilisés depuis longtemps, sous forme réticulée, comme matrice à l'intérieur de laquelle est dispersé le principe actif ou comme matière constitutive de la capsule, c'est-à-dire du revêtement encapsulant le principe actif.

Il existe de très nombreux brevets décrivant des systèmes matriciels (par exemple US-A-4 053 580 et FR-A-2 560 768) et des systèmes par encapsulation (par exemple EP-A-171 907, US-A-4 011 312 et US-A-4 273 920).

Dans ce type d'application les silicones ne sont pas dégradables par hydrolyse et ne sont donc pas érodables.

De plus, la réticulation de ces polymères silicones se fait en présence du principe actif. Cette réticulation se fait généralement au moyen d'un catalyseur organométallique de durcissement, d'un chauffage, ou d'un rayonnement (ultra-violet, infra-rouge, faisceau d'électrons, gamma), ou par une combinaison de ces moyens. Cette réticulation faisant intervenir des réactions chimiques ou photochimiques en présence du principe actif, peut donc avoir une action néfaste sur ce principe actif.

Par ailleurs, les propriétés physicochimiques intrinsèques du principe actif peuvent perturber, voir inhiber complètement le processus de réticulation.

En outre dans les applications pharmaceutiques et biologiques, le caractère biocompatible du polymère a une importance fondamentale.

Pour ces applications il est très difficile, parfois impossible, d'éliminer hors du polymère silicone réticulé des produits indésirables tels que catalyseurs et polymères silicones résiduels non intégrés au réticulat. Enfin l'utilisation de silicone réticulé non dégradable interdit la libération d'un principe actif macromoléculaire.

L'utilisation de polyesters dégradables du type polylactique, polyglycolique et leurs copolymères a été tout d'abord décrite pour la réalisation de fils de suture chirurgicale biodégradables (US-A-2 703 316, US-A-2 758 987 et FR-A-1 425 333).

Ces polyesters ont été décrits également comme matrice pour la libération contrôlée de principe actif (EP-A-171 907, US-A-4 011 312 et US-A-4 273 920).

Pour les applications de libération contrôlée les polyesters dégradables présentent des avantages importants :

- ils sont non toxiques ainsi que leurs produits de dégradation engendrés par l'hydrolyse,
- on peut adapter dans une certaine mesure la durée, le profil de libération du principe actif, la cinétique d'hydrolyse, notamment par le choix des monomères de départ, par la longueur de la chaîne, sa cristallinité, etc .....,

Ils présentent toutefois une température de transition vitreuse trop élevée pour permettre la diffusion d'un grand nombre de principes actifs.

Ils nécessitent souvent des températures de mise en oeuvre élevées qui peuvent être incompatibles avec la stabilité thermique d'un grand nombre de principes actifs.

Le but de la présente invention est de proposer un copolymère dégradable par hydrolyse, érodable, thermoplastique pouvant présenter des propriétés mécaniques suffisantes et de mise en forme aisée.

Un autre but de la présente invention est de proposer un copolymère qui présente à la fois les propriétés avantageuses des silicones et des polyesters dégradables sans en présenter les inconvénients, ou tout au moins, sous une forme très atténuée.

Ce but est atteint par la présente invention qui concerne en effet un copolymère à bloc diorganopolysiloxane-polyester présentant de 1 à 99 %, de préférence de 10 à 90 %, en poids de segment diorganopolysiloxane de formule moyenne :

$$- Y - \left[ \begin{array}{c} R \\ SiO \\ R \end{array} \right]_m \begin{array}{c} R \\ Si \\ R \end{array} - Y - \qquad (1)$$

et de 99 à 1 % en poids de segment polyester de formule moyenne :

$$\left[ \begin{array}{c} -CH - C - O - \\ \;\; | \quad\;\; \| \\ \;\; H \quad\;\; O \end{array} \right]_{p2} \left[ \begin{array}{c} -CH - C - O - \\ \;\; | \quad\;\; \| \\ \;\; CH_3 \;\; O \end{array} \right]_{p1} - Z - \left[ \begin{array}{c} -O - C - CH - \\ \quad\;\; \| \quad\; | \\ \quad\;\; O \;\; CH_3 \end{array} \right]_{r1} \left[ \begin{array}{c} -O - C - CH - \\ \quad\;\; \| \quad\; | \\ \quad\;\; O \quad\; H \end{array} \right]_{r2} \qquad (2)$$

les segments (1) et (2) étant reliés entre eux par un pont organique ou organosilicié présentant à chaque extrémité une liaison uréthanne.

Dans la formule (1), les radicaux R, identiques ou différents, sont des radicaux organiques monovalents. Ils sont choisis de préférence parmi les radicaux alkyle en $C_1$-$C_6$, phényle, vinyle et trifluoro-3,3,3 propyle.

Les motifs diorganosiloxyle préférés de par leur disponibilité industrielle sont les motifs diméthylsiloxy, méthylphénylsiloxy et diphénylsiloxy.

m est un nombre entier ou fractionné compris entre 1 et 500, de préférence entre 5 et 200,

Y représente un radical organique divalent relié à l'atome de silicium par une liaison SiC,

Dans la formule du segment (2), $p_1$, $p_2$, $r_1$ et $r_2$ sont des nombres entiers ou fractionnés compris entre 0 et 10 000 inclus avec :

$r_1 + r_2 + p_1 + p_2$ supérieur à 1 et inférieur à 10 000, de préférence compris entre 10 et 200 inclus.

Z est un radical hydrocarboné, divalent de formule :

$- CH_2 - W - CH_2 -$,

dans laquelle W est un radical hydrocarboné, saturé ou insaturé, divalent linéaire, ramifié ou cyclique ayant de 1 à 8 atomes de carbone, ou bien symbolise une liaison covalente.

A l'intérieur du segment polyester de formule (2) les séquences lactique et glycolique peuvent être distribuées de façon statistique, alternée ou à blocs.

Les teneurs en poids des segments (1) et (2) sont calculés par rapport au poids total des segments (1) et (2).

Le pont liant les segments (1) et (2) répond à la formule :

$$- O - \overset{\displaystyle O}{\overset{\|}{C}} - NH - B - NH - \overset{\displaystyle O}{\overset{\|}{C}} - O - \qquad (3)$$

où B est un radical organique ou organosilicié divalent pouvant éventuellement comporter un segment diorganopolysiloxane de formule moyenne :

$$- \left[ \begin{array}{c} R^1 \\ SiO \\ R^1 \end{array} \right]_n \begin{array}{c} R^1 \\ Si \\ R^1 \end{array} - \qquad (1\ bis)$$

dans laquelle $R^1$ a la même signification que R et n a la même signification que m données ci-dessus, pour la formule (1). Bien entendu dans le cas où le copolymère selon l'invention comporte à la fois des segments de formules (1) et (1 bis), R et $R^1$, m et n peuvent avoir des significations identiques ou différentes.

Les copolymères selon l'invention sont préparés à partir des produits de départ a), b) et c) suivants :

a) - un oligomère diorganopolysiloxane de départ répondant à la formule générale moyenne :

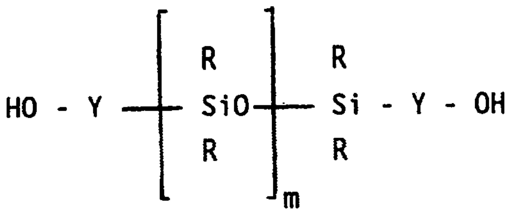

(4)

dans laquelle les symboles Y, identiques ou différents, représentent un radical organique divalent relié à l'atome de silicium par une liaison SiC et R et m a la même signification que ci-dessus.

De préférence, le chaînon Y représente un chaînon alkylène linéaire ou ramifié en $C_1$-$C_{18}$ inclusivement, éventuellement prolongé par un chaînon polyéther choisi parmi le polyoxyde d'éthylène, le polyoxyde de propylène et leurs mélanges et comportant de 1 à 50, de préférence de 5 à 30 motifs oxyde d'éthylène et/ou oxyde de propylène.

Comme exemples de chaînons Y, on peut citer :

$- CH_2 -$, $-(CH_2)_2 -$, $-(CH_2)_3-$, $(OCH_2 - CH_2)_{29}$, $-(CH_2)_3-$, $- (CH_2)_3-\{O - CH_2 - CH(CH_3)\}_{15}-$, $-(CH_2)_3 - O -(CH_2 - CH_2)-$, $- (CH_2) - CH(CH_3) - CH_2 -$, $-(CH_2)_{12}-$.

b) - un oligomère polyester de départ répondant à la formule générale moyenne :

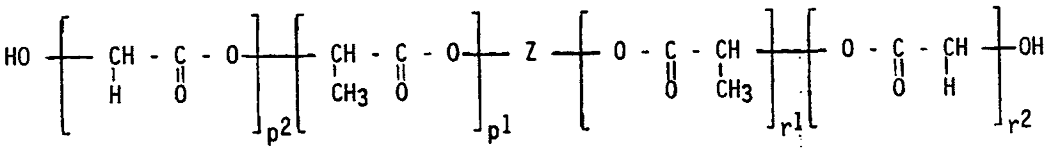

(5)

dans laquelle Z, $p_1$, $p_2$, $r_1$ et $r_2$ ont la même signification qu'à la formule (2) ci-dessus.

W est donc une simple liaison covalente, ou un chaînon alkylène ou cycloalkylène comme par exemple :

$- CH_2 -$, $- (CH_2)_2 -$, $- CH(CH_3)-$,

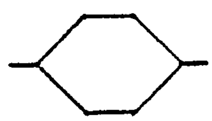

Les procédés d'obtention du polyester de formule (5) sont de deux types : par polycondensation ou par polymérisation par ouverture de cycle.

Les procédés de ce type sont décrits dans les brevets US-A-2 676 945, US-A-4 273 920, FR-A-1 425 333, FR-A-2 086 047, EP-A-171 907 et EP-A-172 636.

Selon le procédé de polycondensation on place l'acide lactique, l'acide glycolique ou un mélange des deux monomères dans un réacteur fermé en présence du diol de formule $HO - CH_2 - W - CH_2 - OH$.

On effectue la polycondensation, de préférence sans catalyseur en augmentant la température et en diminuant la pression simultanément. Le temps de réaction peut aller par exemple de 5 à 120 heures ; on peut faire passer la température de 20 à 220 °C et abaisser simultanément la pression de la pression atmosphérique à 0,02 KPa ou moins.

Selon le procédé de polymérisation recommandé dans le cadre de la présente invention, on utilise des dimères cycliques des acides lactiques ou glycoliques nommés lactides ou glycolides ou leurs mélanges en présence du diol.

Le lactide utilisé peut être du lactide L(-) optiquement pur ou du lactide DL racémique.

Ceci conditionne la stéréorégularité du polylactique obtenu. Ainsi dans le cas du lactide L(-) on obtient un polymère semi-cristallin, alors qu'avec du lactide DL, on obtient un polymère amorphe.

On effectue la polymérisation en masse en présence d'un catalyseur.

Les conditions de durée de réaction, de température et de pression sont les suivantes :

- temps de réaction compris entre 3 et 120 heures,
- température comprise entre 110 et 180 °C et,
- pression inférieure à 0,02 KPa.

Les caractéristiques de poids moléculaire, de polydispersité et de cristallinité du polymère obtenu selon l'un ou l'autre procédé, sont contrôlées par les conditions expérimentales et la composition des monomères de départ.

c) - un composé diisocyanato de formule :

$$O = C = N - B - N = C = O \quad (6)$$

dans laquelle B symbolise un radical organique ou organosilicié divalent pouvant comporter un segment de formule (1 bis) ci-dessus.

Dans le cas où B est un radical organique divalent il comporte de 3 à 30 atomes de carbone, de préférence de 4 à 20 atomes de carbone.

Comme composés organiques diisocyanato on peut en particulier utiliser :

- le diisocyanato-1,2 propane,
- le diisocyanato-1,2 butane,
- le diisocyanato-1,3 butane,
- le diisocyanato-1,6 hexane,
- le diisocyanato-1,3 benzène,
- le diisocyanato-1,4 benzène,
- le diisocyanato-2,4 toluène,
- le diisocyanato-2,6 toluène,
- le diisocyanato-2,4 xylène,
- le diisocyanato-2,6 xylène,
- le diisocyanato-3,3' biphényle,
- le diisocyanato-4,4' biphényle,
- le diisocyanato-3,3' diphénylméthane,
- le diisocyanato-4,4' diphénylméthane,
- le diisocyanato-4,4' diméthyl-3,3' diphényle,
- le diisocyanato-4,4' diméthyl-3,3' diphénylméthane,
- le diisocyanato-4,4' diphényléthane,
- le diisocyanato-3,3' diphényléther,
- le diisocyanato-4,4' diphényléther,
- le diisocyanato-3,3' diphénylsulfone,
- le diisocyanato-4,4' diphénylsulfone,
- le diisocyanato-3,3' benzophénone,
- le diisocyanato-4,4' benzophénone,
- le diisocyanato-3,3' dicyclohexylméthane,
- le diisocyanato-4,4' dicyclohexylméthane,
- le diisocyanato-1,5 naphtalène,
- le diisocyanato-4,4' dichloro-3,3 biphényle,
- le diisocyanato-4,4' diméthoxy-3,3' biphényle

Les diisocyanates utilisés préférentiellement sont les suivants :

- le diisocyanato-1,6 hexane,
- le diisocyanato-2,4 toluène,
- le diisocyanato-2,6 toluène,
- le diisocyanato-2,4 xylène,
- le diisocyanato-4,4' biphényle,
- le diisocyanato-4,4' diphénylméthane,
- le diisocyanato-4,4' diphényléther,
- le diisocyanato-4,4' diphénylsulfone,
- le diisocyanato-4,4' benzophénone,
- le diisocyanato-4,4' dicyclohexyléthane,
- le diisocyanato-1,5 naphtalène.

Le copolymère selon l'invention est obtenu par polyaddition, de préférence au sein d'un solvant organique, des produits de départ a), b) et c) en des quantités telles que le rapport molaire des groupes isocyanates sur des groupes OH soit compris entre 0,95 et 1,05.

Les solvants préférés sont les hydrocarbures halogénés aliphatiques ou aromatiques. Parmi ceux-ci il est recommandé d'utiliser l'o-dichlorobenzène ou le tétrachloroéthane. La réaction de polyaddition peut être effectuée en absence ou en présence de catalyseur. Comme catalyseurs recommandés on peut citer les dicarboxylates de dialkylétain comme le dilaurate de dibutylétain.

La réaction de polyaddition est effectuée par simple chauffage des réactifs à une température en

général comprise entre 100 et 180 °C.

Selon un mode de réalisation préféré on introduit le polyester b) dans une fraction du solvant que l'on porte à une température comprise entre 100 et 180 °C, on ajoute le catalyseur et les produits de départ a) et c) avec le reste du solvant. On contrôle la fin de la réaction par dosage chimique des fonctions isocyanates résiduelles. On récupère le copolymère après élimination du solvant.

Dans le cas où dans le composé (6), B est un radical organosilicié, B peut comporter un segment diorganosiloxane de formule (1 bis). Dans ce cas, le copolymère selon l'invention peut ne pas comporter de segment diorganopolysiloxane de formule (1).

Les composés (6) comportant un segment de formule (1 bis) peuvent être obtenus par réaction d'hydrosilylation entre un α,ω-(dihydrogéno)polydiorganosiloxane sur un composé oléfinique porteur d'un groupement isocyanate. De tels composés (6) sont en particulier décrits dans EP-A-77 744 cité comme référence.

Lorsque le composé (6) contient un segment diorganopolysiloxane, il n'est donc pas obligatoire d'utiliser le produit de départ a).

Dans ce cas on recommande d'utiliser le procédé suivant pour préparer les copolymères à blocs alternés selon l'invention :

- au cours d'une première étape on fait réagir l'oligomère polyester b) de formule (5) sur un excès molaire d'un composé oléfinique porteur d'un groupement isocyanate en particulier décrit dans le brevet européen EP-A-77 744, de préférence répondant à la formule générale :

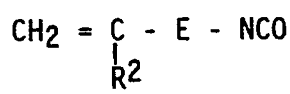 (7)

dans laquelle $R^2$ est choisi parmi un atome d'hydrogène et un radical alkyle en $C_1$-$C_6$, E est un radical hydrocarboné exempt d'insaturation oléfinique, ayant de 1 à 20 atomes de carbone, et pouvant comporter au moins un hétéroatome choisi parmi O et Si, de préférence au sein d'un solvant organique en présence d'un catalyseur à l'étain tel que le dilaurate de dibutylétain.

Les composés de formule (7) les plus préférés répondent à la formule :

$CH_2 = CH - CH_2 - NCO$

$CH_2 = C(CH_3) - CH_2 - NCO$

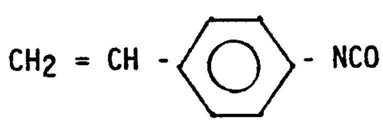

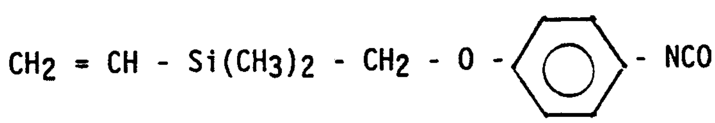

- au cours d'une deuxième étape on fait réagir l'oligomère polyester (bis-α,ω vinyle) obtenu au cours de la première étape sur un diorganopolysiloxane de formule :

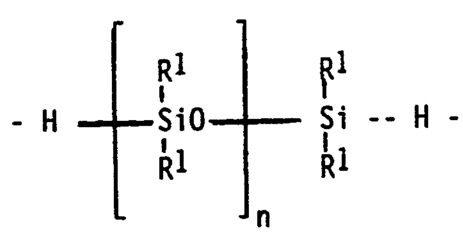 (8)

dans laquelle $R^1$ et n ont la signification donnée à la formule (1 bis) ci-dessus, en présence d'une quantité catalytiquement efficace d'un catalyseur d'hydrosilylation, de préférence un catalyseur au platine suivant un rapport molaire SiH compris entre 0,9 et 1,5.

$$CH_2 = \underset{R^2}{\underset{|}{C}} -$$

Les catalyseurs au platine utilisés pour réaliser la réaction d'hydrosilylation des polymères de formule (8) sur le dérivé de formule (7) sont amplement décrits dans la littérature, on peut en particulier citer les complexes du platine et d'un produit organique décrit dans les brevets américains US-A-3 159 601, US-A-3 159 602, US-A-3 220 972 et les brevets européens EP-A-57 459, EP-A-188 978 et EP-A-190 530 et les complexes du platine et d'organopolysiloxane vinylé décrits dans les brevets américains US-A-3 419 593, US-A-3 715 334, US-A-3 377 432 et US-A-3 814 730.

Pour faire réagir le polymère à SiH de formule (8) sur le dérivé de formule (7) on utilise généralement une quantité de catalyseur au platine calculée en poids de platine métal comprise entre 5 et 600 ppm, de préférence entre 10 et 200 ppm basés sur le poids de polymère à SiH de formule (8).

La réaction d'hydrosilylation peut avoir lieu en masse ou au sein d'un solvant organique volatil tel que le toluène, l'heptane, le xylène, le tétrahydrofuranne et le tétrachloroéthylène.

Il est généralement souhaitable de chauffer le mélange réactionnel à une température de 60 à 120 °C pendant le temps nécessaire pour que la réaction soit complète. Par ailleurs il est souhaitable d'ajouter goutte-à-goutte le polymère à SiH sur le dérivé de formule (7) en solution dans un solvant organique.

On vérifie le degré d'avancement de la réaction en dosant les SiH résiduels par la potasse alcoolique puis on élimine le solvant par exemple par distillation sous pression réduite.

L'huile brute obtenue peut être purifiée, par exemple par passage sur une colonne absorbante de silice.

Les copolymères selon l'invention sont thermoplastiques et dégradables par hydrolyse et peuvent constituer tout ou partie d'objets dégradables par hydrolyse.

Ils présentent des propriétés mécaniques suffisantes pour se présenter à température ambiante sous forme d'objets dégradables par hydrolyse de formes quelconques contenant un principe actif, à une teneur généralement comprise entre 0,1 et 40 % en poids, qui peut être notamment un principe actif médicamenteux, un produit phytosanitaire (tel qu'un engrais, un insecticide, un pesticide, un fongicide, un herbicide), un embryon végétal, une semence végétale, un catalyseur, un produit cosmétique, etc.....

Dans le cas où le principe actif est encapsulé, cette teneur peut aller de 0,1 à 99 % en poids suivant le procédé d'encapsulation utilisé.

Parmi les principes actifs médicamenteux peuvent être cités :

- les agents antiinflammatoires tels que :
. le kétoprofène,
. l'ibuprofène,
. l'indométhacine,
- les agents hormonaux tels que :
. les stéroïdes,
. les hormones peptidiques,
- les agents antitumoraux,
- les antibactériens tels que :
. les pénicillines,
. les céphalosporines,
. les streptomycines.

Les copolymères selon l'invention présentent donc des propriétés mécaniques suffisantes pour des applications en libération contrôlée. Ils sont en outre très faciles à mettre en forme à basse température. Ils peuvent être utilisés pour la libération contrôlée de principes actifs macromoléculaires, ce qui n'est pas possible au moyen de simples polymères silicones.

Un autre avantage du copolymère, selon l'invention, est qu'il permet d'encapsuler un principe actif par pelliculages successifs en utilisant un procédé comprenant au moins une étape de pulvérisation d'une composition pelliculante contenant le copolymère en solution dans un solvant organique ou sous la forme d'une émulsion ou d'une dispersion aqueuse suivie d'au moins un séchage, c'est-à-dire de l'évaporation du solvant organique et/ou de l'eau. Dans ce cas la teneur en principe actif de la capsule peut aller de 40 à 99 % en poids par rapport au poids total de l'objet.

Un exemple d'un tel procédé est le procédé connu sous l'appellation "spray coating" selon lequel les particules à encapsuler sont brassées (fluidisées) par un flux gazeux qui assure également le sèchage, c'est-à-dire l'évaporation du solvant organique et/ou de l'eau.

La composition pelliculante est pulvérisée par une ou plusieurs buses situées à différentes zones du

réacteur suivant le type de procédé utilisé par exemple, au-dessus, à l'intérieur ou à la base du nuage de particules.

Ainsi cette pulvérisation est à la base du lit fluidisé de particules dans le procédé WURSTER. La technique de pulvérisation de WURSTER est décrite en détails dans les brevets US-A-2 799 241, US-A-3 089 824, US-A-3 117 027, US-A-3 196 827, US-A-3 207 824, US-A-3 241 520, US-A-3 523 994 et EP-A-188 953.

Selon d'autres procédés utilisables dans le cadre de la présente invention, le brassage des particules de principe actif est effectué mécaniquement, par exemple au moyen d'un tambour ou d'un plateau rotatif et le courant gazeux ne sert qu'au séchage.

Pour obtenir une encapsulation efficace, il est recommandé que la pellicule de revêtement présente une épaisseur moyenne comprise entre 1 et 200 µm, de préférence entre 5 et 100 µm.

Bien ententu l'homme de métier, par des essais de routine, peut adapter sans difficulté l'épaisseur du revêtement à la nature du principe actif, à son état de surface, à la nature du copolymère utilisé, à la cinétique désirée de libération du principe actif, etc.....

Il est possible également d'encapsuler les particules par exemple par une première capsule en copolymère selon l'invention puis de réaliser une deuxième couche de nature différente et par un procédé différent, par exemple d'une cire naturelle ou synthétique.

Les principes actifs médicamenteux encapsulés par lesdits copolymères font aussi partie de l'invention. Ils présentent une cinétique de libération très intéressante lorsqu'ils sont administrés à l'homme.

La plupart des médicaments ont une profil d'acitivté "in vivo" et pour que ce profil soit atteint il demande une formulation pharmaceutique spécifique, particulièrement lorsque l'on recherche une concentration plasmatique soigneusement contrôlée. La disponibilité du médicament sur de longues périodes et la facilité du traitement ambulatoire apportent aussi des exigences en ce qui concerne la nature des excipients de formulation. Chaque malade préfère absorber son médicament une fois par jour plutôt que plusieurs fois sur la même période. En plus, la libération du principe actif doit se faire la plus régulièrement possible de façon, surtout pour les analgésiques, à éviter au malade les crises douloureuses pendant les latences où le principe actif n'est pas actif. Les principes actifs encapsulés de l'invention répondent à cet objectif.

Les exemples suivants illustrent l'invention :

GPC signifie chromatographie par perméation gazeuse,
DSC signifie calorimétrie différentielle,
Mn signifie masse moléculaire moyenne en nombre,
Mw signifie masse moléculaire moyenne en poids,
Ip signifie indice de polydispersité.
La résistance à la rupture R/R est mesurée en MPa selon la norme AFNOR-T 46 002.
L'allongement à la rupture A/R est mesurée en selon la norme AFNOR-T 46 002.
Tg est la température de transition vitreuse.
Tm est la température de fusion.
$\phi$ représente le radical phényle.
PLA DL : polylactique racémique.

On fera référence au dessin annexé sur lequel la figure 1 donne, en ordonnée, le % de ketoprofène libéré en fonction du temps (en abscisse et en jours) pour les exemples 13 et 14, la figure 2 donne le % de nicergoline libérée pour les exemples 16, 17 et 18 et la figure 3 donne le % de bovalbumine libéré pour les exemples 19, 20 et 21.

- EXEMPLE 1 :

1a. - Préparation d'un polyester de formule :

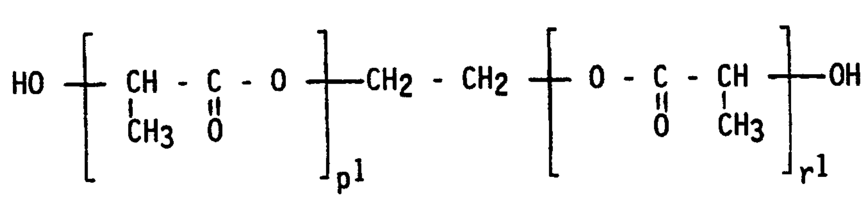

On charge dans un réacteur de 1 litre, 291 g de lactide L(-) fraîchement recristallisé dans l'acétate d'éthyle, 8,2 ml d'éthylèneglycol distillé sous vide et 0,510 g d'octanoate d'étain. On établit un vide inférieur à 0,02 KPa dans le réacteur puis on chauffe à 120 °C pendant 48 heures.

Après refroidissement, le produit réactionnel est dissout dans 500 ml de $CH_2Cl_2$ puis purifié en coulant cette solution dans de l'eau à 65 °C sous forte agitation. Après évaporation du solvant, on récupère 268 g de polymère séché sous vide présentant les caractéristiques suivantes :

Tg : 30 °C,
Tm : 78 °C,
Mn : 2 010,
Teneur en fonction OH : 0,97 équivalent par Kg.

<u>1b . - Préparation du pont diisocyanate de formule : ABA</u>

avec A :

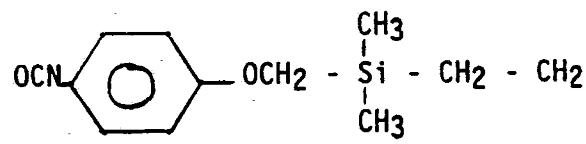

et B :

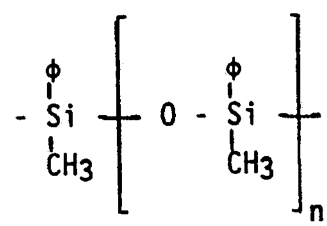

Dans un réacteur en verre de 500 ml équipé d'un agitateur et d'un réfrigérant ascendant, on charge :
- 40 ml de toluène,
- 38,7 g (0,166 mole) d'un isocyanato silane de formule :

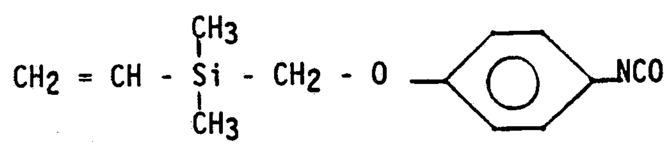

dont la préparation est décrite à l'exemple 8 de US-A-4 088 670.
- 10 mg (calculés en platine métal) pour 1 kg de réactif, d'acide chloroplatinique en solution dans l'éthyl-2 hexanol.

Le mélange est agité et chauffé à 70 °C et on coule pendant 35 minutes, 75 g d'un α,ω-(dihydrogéno)-polyméthylphénylsiloxane de formule :

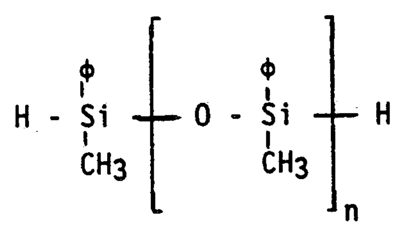

Mn = 900

en solution dans le toluène. Le mélange est ensuite agité et chauffé à 90 °C pendant 4 heures. Ensuite la

masse réactionnelle est dévolatilisée à 120 °C sous pression réduite (0,07 KPa).

Le fluide obtenu est limpide et incolore. Un dosage chimique des fonctions isocyanate permet de calculer une masse Mn de 1 515 g/mole, soit 1,32 équivalent de fonction isocyanate/Kg.

1c. - Préparation du copolymère :

Dans un réacteur en verre de 100 ml équipé d'un agitateur et d'un réfrigérant ascendant, on charge : 15,2 g d'oligomère obtenu à l'exemple 1b., 20,6 g du polyester obtenu à l'exemple 1a. et 45 ml d'o-dichlorobenzène.

Le polyester est chargé en premier avec une partie du solvant. Le mélange est agité et chauffé jusqu'à l'obtention d'une solution homogène. Ensuite, l'huile silicone, préalablement diluée avec le reste du solvant, est ajoutée au mélange. Puis on introduit 18 mg de dilaurate de di-n-butylétain pour catalyser la réaction. On maintient l'agitation à 125 °C pendant 5 heures 10 minutes. Par dosage chimique on vérifie que le milieu réactionnel ne contient plus de fonctions isocyanate détectables. Le solvant est éliminé dans un évaporateur rotatif à 130 °C sous une pression de 0,133 KPa.

On obtient 34,35 g de copolymère que l'on redissout dans 40 ml de dichlorométhane et que l'on précipite dans 500 ml d'hexane refroidi à 3,5 °C.

Le copolymère est séparé puis séché à l'étuve à 100 °C sous pression réduite.

Le produit sec est rebroyé pour donner une poudre fine qui donne un solide rigide et transparent quand on le moule par compression.

La quantité de poudre obtenue représente une masse de 30,8 g.

Caractérisation du copolymère purifié :

- viscosité inhérente (dans le chloroforme à 25 °C, C = 3g/dl) : 0,17 dl/g,
- masses moléculaires (par GPC, étalonnage polystyrène) :
Mn : 4 620 g/mole ; Mw : 16 400 g/mole,
- température de transition vitreuse (par DSC à 10 °C/mn après un premier chauffage à 180 °C : Tg = 30 °C.

- EXEMPLE 2 :

Dans un réacteur en verre de 100 ml, équipé d'un agitateur et d'un réfrigérant ascendant, on charge :

- 10,3 g du polyester de l'exemple 1a.,
- 5,4 g d'huile polyméthylphénylsiloxane fonctionnalisée de formule :

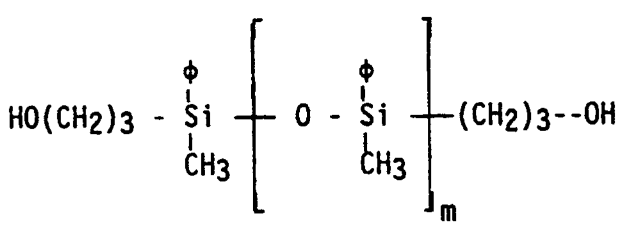

présentant une teneur en fonction OH égale à 1,84 équivalent/kg,

- 1,7 g de diisocyanato-1,6 hexane de formule :
$OCN-(CH_2)_6-NCO$
- 25 ml d'o-dichlorobenzène.

Le diol polylactique est chargé en premier avec une partie du solvant. Le mélange ainsi formé est agité et chauffé à l'aide d'un bain d'huile jusqu'à dissolution complète du polyester et obtention d'une solution homogène. Ensuite l'huile silicone fonctionnalisée et le diisocyanato-1,6 hexane préalablement mélangés et dilués avec le reste du solvant, sont ajoutés à leur tour dans le réacteur.

Enfin on catalyse le mélange en introduisant 12 mg de dilaurate de dibutylétain.

Après 6 heures 15 minutes d'agitation à 120 °C, on vérifie par un dosage chimique que le milieu réactionnel ne contient plus de fonctions isocyanate détectables.

Après élimination du solvant dans un évaporateur rotatif (à 130 °C, sous une pression de 0,133 KPa), on obtient 17,3 g d'un matériau presque incolore, souple et transparent.

Caractérisations :
- viscosité inhérente (dans le chloroforme, à 25 °C, C = 3 g/dl) : 0,30 dl/g
- masses moléculaires (par GPC, étalonnage polystyrène) :
Mn = 4 870 g/mole Mw = 38 210 g/mole
- température de transition vitreuse (par DSC) : 10 → 30 °C
- température de ramollissement : 60/70 °C (banc de Kofler)
- propriétés mécaniques à 20 °C : le copolymère a été placé dans un moule et comprimé à 100 KPa, à 80 °C. On a obtenu une plaque moulée de 1 mm d'épaisseur dans laquelle on a découpé des éprouvettes de type $H_3$.

Les mesures des propriétés mécaniques ont été réalisées avec un appareil de type INSTRON 1 026 sur 9 éprouvettes $H_3$ :
- résistance à la rupture : 1,17 ± 0,25 MPa,
- allongement à la rupture : 360 ± 76 %.

- EXEMPLE 3 :

Dans un réacteur en verre de 100 ml équipé d'un agitateur et d'un réfrigérant ascendant, on charge :
- 10,3 g de polyester obtenu à l'example 1a.
- 13,0 g d'un oligomère polydiméthylsiloxane fonctionnalisé, répondant à la formule suivante :

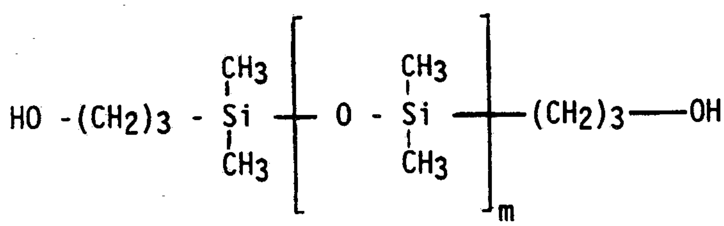

et caractérisé par une teneur en fonction OH égale à : 0,77 équivalent par kg.
- 1,7 g de diisocyanato-1,6 hexane,
- 27 ml d'o-dichlorobenzène.

Le diol polylactique est chargé en premier avec une partie du solvant. Le mélange est agité et chauffé à l'aide d'un bain d'huile jusqu'à dissolution du polyester et obtention d'une solution homogène.

Ensuite, l'oligomère polysiloxane et le diisocyanate préalablement mélangés et dilués avec le reste du solvant, sont introduits à leur tour dans le réacteur.

Enfin, on ajoute 12 mg de dilaurate de dibutylétain (catalyseur). On maintient un chauffage à 120 °C, sous agitation, pendant 6 heures 15 minutes.

Après avoir vérifié que le milieu réactionnel ne contient plus de fonctions isocyanate détectables, on évapore le solvant à l'évaporateur rotatif (130 °C, 0,133 KPa).

On obtient ainsi 22,4 g d'un matériau légèrement coloré, opaque, très souple (élastomère).

Caractérisations :
- viscosité inhérente (dans le chloroforme, à 25 °C, C = 3 g/dl) : 0,33 dl/g
- masses moléculaires (par GPC, étalonnage polystyrène) :
Mn = 4 330 g/mole ; Mw = 44 770 g/mole
- température de ramollissement : environ 60 °C (au banc Kofler).

- EXEMPLE 4 :

Dans un réacteur en verre de 100 ml équipé d'un agitateur et d'un réfrigérant ascendant, on charge :
- 15,79 g du polyester préparé à l'exemple 1a.
- 8,25 g d'un polyméthylphénylsiloxane fonctionnalisé répondant à la formule :

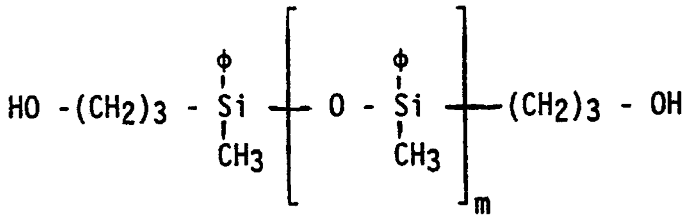

et caractérisé par une teneur en fonctions hydroxyle de : 1,82 équivalent par kg,
- 2,55 g de diisocyanato-1,6 hexane,
- 30 g d'ortho-dichlorobenzène.

Le polyester est chargé en premier dans le réacteur, avec une partie du solvant. Le mélange ainsi formé est agité et chauffé à l'aide d'un bain d'huile thermostaté. On obtient une solution homogène.

On ajoute ensuite le polysiloxane fonctionnalisé et le diisocyanato-1,6 hexane, tous deux préalablement mélangés et dilués avec le reste des 30 g de solvant.

Enfin, la température du milieu réactionnel étant d'environ 110 °C, on ajoute 0,0132 g de dilaurate de dibutylétain (catalyseur).

L'avancement de la réaction est suivi par dosage des fonctions isocyanate.

Après deux heures de chauffage à 120 °C, le milieu réactionnel est placé dans un évaporateur rotatif à 130-140 °C sous une pression de 0,133 KPa environ, pour l'élimination du solvant.

Le produit obtenu est dissout dans du dichlorométhane, précipité dans de l'hexane et séché à l'étuve pendant 15 heures.

On obtient 21,0 g d'un matériau faiblement coloré, transparent et thermoplastique (assez rigide).

Caractérisations :
- viscosité inhérente (dans le chloroforme, à 25 °C, C = 1,5 g/dl) : 0,49 dl/g,
- masses moléculaires (par GPC, étalonnage polystyrène) :
Mn = 7 550 g/mole ; Mw = 61 720 g/mole,
- température de ramollissement : 60-70 °C (au banc de Kofler),
- propriétés mécaniques à 20 °C :
le produit a été placé dans un moule chauffé à 170 °C et comprimé à 80 MPa.

On a obtenu ainsi une plaque moulée de 0,5 mm d'épaisseur dans laquelle on a découpé des éprouvettes de type $H_3$.

Les propriétés mécaniques ont été déterminées à l'aide d'un appareil de type INSTRON 1 026 :
- résistance à la rupture : 9,7 MPa,
- allongement à la rupture : 87 %.

## - EXEMPLE 5 :

Dans un réacteur en verre de 100 ml équipé d'un agitateur et d'un réfrigérant ascendant, on charge :
- 6,27 g du polyester préparé à l'exemple 1a.
- 13,21 g de l'oligomère polyméthylphénylsiloxane fonctionnalisé répondant à la formule :

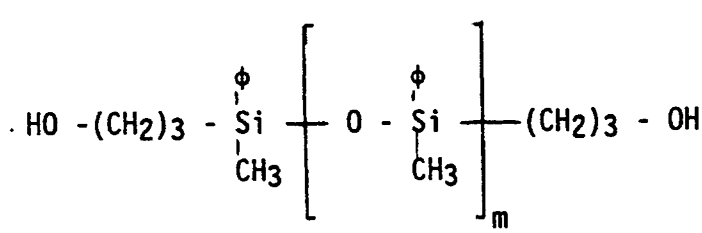

et caractérisé par une teneur en fonctions hydroxyle de : 1,82 équivalent par kg,
- 2,55 g de diisocyanato-1,6 hexane,
- 25 g d'ortho-dichlorobenzène.

Le polyester est chargé en premier dans le réacteur, avec une partie du solvant. Le mélange ainsi formé est agité et chauffé à l'aide d'un bain d'huile thermostaté. On obtient une solution homogène.

Ensuite, le polysiloxane fonctionnalisé et le diisocyanato-1,6 hexane, préalablement mélangés et dilués avec le reste du solvant, sont introduits à leur tour dans le réacteur.

Puis, alors que la température du mélange est d'environ 110 °C, on ajoute 0,010 g de dilaurate de dibutylétain (catalyseur).

L'avancement de la réaction est suivi par dosage des fonctions NCO.

Le milieu réactionnel est ainsi agité à 120 °C pendant 1 heure 30 minutes.

Ensuite le solvant est éliminé dans un évaporateur rotatif à 130-140 °C sous une pression de 0,133 KPa.

Le polymère est ensuite redissout dans 25 ml de dichlorométhane et précipité, sous forte agitation, dans 250 ml d'hexane.

Le copolymère recueilli est ensuite séché à l'étuve, sous pression réduite (13,3 KPa), à 50 °C.

On obtient ainsi 19,9 g de produit sec.

C'est un matériau faiblement coloré, transparent, très souple et thermoplastique.

Caractérisations :

- viscosité inhérente (dans le chloroforme, à 25 °C, C = 1,5 g/dl) : 0,27 dl/g,
- masses moléculaires (par GPC, étalonnage polystyrène) :

Mn = 7 570 g/mole ; Mw = 33 890 g/mole,

- propriétés mécaniques à 20 °C :

le produit a été placé dans un moule chauffé à 150 °C et comprimé à 80 MPa.

On a obtenu ainsi une plaque moulée de 0,8 mm d'épaisseur dans laquelle on a découpé des éprouvettes de type $H_3$.

Les propriétés mécaniques ont été déterminées à l'aide d'un appareil de type INSTRON 1 026 :

- résistance à la rupture : 0,4 NPa,
- allongement à la rupture : 1 375 %.

## - EXEMPLE 6 :

On reproduit exactement le mode opératoire de l'exemple 5, sauf que l'on charge dans le réacteur :

- diol polylactique : 10,75 g
- polyméthylphenylsiloxane fonctionnalisé NCO : 10,88 g
- diisocyanato-1,6 hexane : 2,55 g
- ortho-dichlorobenzène : 30 g
- dilaurate de dibutylétain (catalyseur) : 0,012 g

On obtient 22,2 g de produit sec et reprécipité dans l'hexane.

Caractéristiques du copolymère : mêmes conditions de mesures que pour l'exemple 5 :

- viscosité inhérente (dans le chloroforme, à 25 °C, C = 1,5 g/dl) : 0,30 dl/g,
- masses moléculaires (par GPC, étalonnage polystyrène) :

Mn = 7 610 g/mole ; Mw = 36 660 g/mole,

- propriétés mécaniques à 20 °C :

le produit a été placé dans un moule chauffé à 170 °C et comprimé à 80 MPa.

On a obtenu ainsi une plaque moulée de 0,5 mm d'épaisseur dans laquelle on a découpé des éprouvettes de type $H_3$.

Les propriétés mécaniques ont été déterminées à l'aide d'un appareil de type INSTRON 1 026 :

- résistance à la rupture : 7,5 MPa,
- allongement à la rupture : 212 %.

## - EXEMPLE 7 :

### 7a. - Préparation d'un polyester bis(α,ω-vinyle) :

On charge dans un réacteur de 1 litre, 165 g du polyester préparé à l'exemple 1a., 450 g de toluène et 56 g d'allylisocyanate.

On porte le mélange réactionnel à 70 °C pendant 2 heures 30 minutes et on élimine par chauffage au reflux le solvant et l'allyle isocyanate en excès. Le polymère obtenu (180 g) est dissous dans le dichlorométhane, précipité dans le n-hexane et séché sous vide.

7b. - Préparation d'un copolymère à bloc alterné :

Dans une ampoule de coulée on introduit 35,3 g d'un α,ω-dihydrogénopolysiloxane de formule :

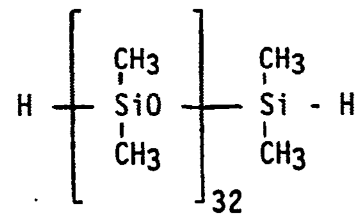

dans 200 g de toluène.

Dans un réacteur de 1 litre on introduit 25 g de polyester bis(α,ω-vinyle) préparé à l'exemple 7a., 200 g de toluène et 49 mg de catalyseur au platine ($H_2PtCl_6$) calculés en platine métal.

On porte la solution dans le réacteur à la température de reflux du toluène tout en y introduisant goutte-à-goutte le contenu de l'ampoule de coulée durant un temps t.

On suit en cours de réaction la disparition des fonctions SiH par spectre I.R. (bande d'absorption caractéristique à 2 100 $cm^{-1}$).

On arrête la réaction lorsque la concentration en fonction SiH reste sensiblement constante.

Le toluène est éliminé dans un évaporateur rotatif à 80 °C sous une pression de 0,133 Kpa.

On obtient 60 g de copolymère à bloc que l'on redissous dans 600 ml de tétrahydrofuranne et que l'on précipite dans 5 000 ml d'eau distillée.

Le copolymère est séparé puis séché à l'étuve à 100 °C sous pression réduite.

Le produit sec est rebroyé pour donner une poudre fine présentant les caractéristiques suivantes :
- Mn = 25 100 g/mole,
- Ip = 2,2.

- EXEMPLES 8 A 11 :

On répète exactement le mode opératoire de l'exemple 7 sauf que l'on modifie la quantité Q (en mg) de catalyseur au platine et/ou la durée t (en h et mn) de la réaction.

Les résultats obtenus sont rassemblés dans le tableau 1 ci-après :

TABLEAU 1

| Exemple | Q (mg) | t (h, mn) | Mn g/mole | Ip |
|---|---|---|---|---|
| 7b. | 49 | 30 mn | 25 100 | 2,2 |
| 8 | 21 | 30 mn | 20 600 | 1,7 |
| 9 | 12 | 1 heure | 15 000 | 1,85 |
| 10 | 6 | 4 heures | 11 000 | 1,6 |
| 11 | 6 | 25 heures | 14 800 | 1,7 |

- EXEMPLE 12 : Synthèse d'un copolymère silicone/lactique : A

Dans un réacteur en verre de 100 ml équipé d'une agitation et d'un réfrigérant, on charge :
- 16,84 g de polylactique fonctionnalisé, répondant à la formule et aux caractéristiques suivantes :

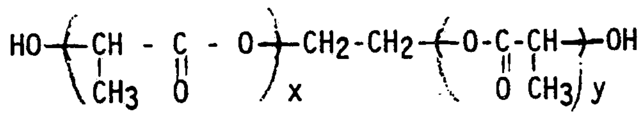

Tg = 15 °C
Mn = 2 000
Teneur en fonctions OH : 0,95 équivalent par Kg
- 10 g de polydiméthylsiloxane fonctionnalisé, répondant à la formule et aux caractéristiques suivantes :

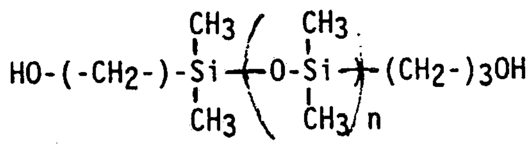

Teneur en fonctions OH : 1,6 équivalent par Kg
- 2,7 g de diisocyanato-1,6 hexane,
- 30 ml d'o-dichlorobenzène.

Le diol polylactique est chargé en premier avec une partie du solvant. Le mélange est agité et chauffé à l'aide d'un bain d'huile jusqu'à dissolution du polyester et obtention d'une solution homogène.

Ensuite, l'oligomère polysiloxane et le diisocyanate préalablement mélangés et dilués avec le reste du solvant, sont introduits à leur tour dans le réacteur.

Enfin, on ajoute 12 mg de dilaurate de dibutylétain (catalyseur). On maintient un chauffage à 120 °C, sous agitation pendat 4 heures 30 minutes.

Après avoir vérifié que le milieu réactionnel ne contient plus de fonctions isocyanate détectables, on évapore le solvant à l'évaporateur rotatif (130 °C, 0,133 KPa).

On obtient ainsi 28,2 g d'un matériau légèrement jaune, transparent, très souple (élastomère).

On dissout le produit obtenu dans 30 ml de chlorure de méthylène et on précipite le polymère dans 300 ml d'hexane à 10 °C. Après récupération et sèchage à l'évaporateur rotatif (35 °C, 0,133 KPa), on récupère 24,6 g de copolymère.

Caractéristiques :
- viscosité inhérente (dans le chloroforme, à 25 °C, C = 3 g/dl) : 0,21 dl/g,
- masses moléculaires (par GPC, étalonnage polystyrène) :
Mn = 6 000 g/mole ; Mw = 22 000 g/mole,
$Tg_1$ = -120 °C ; $Tg_2$ = +15 °C.
($Tg_1$ est relative à la partie silicone et $Tg_2$ à la partie lactique).

- EXEMPLE 13 :

Libération de Kétoprofène® :

On prépare une solution contenant :
- 25 ml de chloroforme,
- 1,7 g de Kétoprofène,
- 4,1 g de copolymère A obtenu à l'exemple 12.

On évapore le solvant et on sèche sous vide. Le produit est ensuite récupéré et compacté à 70 °C pour obtenir un film de 1,5 mm d'épaisseur. Celui-ci est placé dans une enceinte thermostatée à 37 °C contenant 1 litre de solution tamponnée de composition suivante :

| | |
|---|---|
| - $KH_2PO_4$<br>- $H_2O$<br>- NaOH (4N) | 6,8 g<br>1 000 g<br>jusqu'à pH = 7 |

et sous faible agitation.

Le suivi de la cinétique d'élution du Kétoprofène® se fait par dosage UV à 261 nm.

Les résultats obtenus et reportés sur la figure 1 montrent une libération de type diffusionnel pendant 15 jours, suivie d'une libération du reste de Kétoprofène® par érosion du copolymère.

Sur la figure 1 la courbe (1) est relative à l'exemple 13.

- EXEMPLE 14 :

Libération de Kétoprofène® :

On opère comme dans l'exemple 13 mais en remplaçant le copolymère A par un polylactique amorphe PLA-DL de caractéristiques suivantes :
- Mn = 18 300
- Mw = 39 800
- Tg = 45 °C

La libération du Kétoprofène® effectuée dans les conditions de l'exemple 13 montre sur la figure 1 une libération lente du Kétoprofène® au début (temps de latence) qui s'accélère après 15 jours d'élution sous l'effet de l'érosion du polylactique.

Sur la figure 1 la courbe tracée (2) est relative à l'exemple 14.

Ici contrairement à l'exemple 13, on n'observe pas de libération de type diffusionnel.

- EXEMPLE 15 :

Libération de Kétoprofène® :

On opère comme dans l'exemple 13 mais en remplaçant le copolymère A par une composition élastomère silicone réticulable par réaction de polyaddition au platine et commercialisé par la Société RHONE-POULENC sous la dénomination commerciale RHODORSIL± RTV 141.

La présence de Kétoprofène® inhibe la réticulation du silicone et ne permet pas d'obtenir un produit fini à l'état solide après chauffage à 100 °C pendant plusieurs heures.

La comparaison des exemples 13, 14 et 15 montre que le copolymère A permet d'obtenir les avantages du silicone (libération du Kétoprofène® par diffusion) et du polylactique (libération par érosion du matériau), tout en évitant les inconvénients liés aux problèmes de réticulation du silicone et de temps de latence du PLA-DL.

- EXEMPLE 16 :

Libération de Nicergoline® :

On opère de façon identique à l'exemple 13, en remplaçant le Kétoprofène® par la Nicergoline® pour obtenir un film de 1,5 mm d'épaisseur.

On prélève 200 mg de ce film que l'on place dans une enceinte thermostatée à 37 °C, à l'abri de la lumière, contenant 1 litre de solution tamponnée de composition suivante :

| | |
|---|---|
| - $Na_2HPO_4$, 12 $H_2O$ | 8,05 g |
| - $NaH_2PO_4$, $2H_2O$ | 2,03 g |
| - $H_2O$ | 1 000 g |
| - NaOH 4N | jusqu'à pH = 6,5 |

et sous faible agitation.

Le suivi de la cinétique de libération de la Nicergoline® se fait par dosage U.V. à 288 nm.

Les résultats obtenus {courbe (3)} et reportés sur la figure 2 montrent que la libération de la Nicergoline® se fait sur 70 jours de façon complète et à un rythme quasi-constant.

- EXEMPLE 17 :

Libération de Nicergoline® :

On opère comme dans l'exemple 16 mais en remplaçant le copolymère A par un polylactique amorphe PLA-DL ayant les caractéristiques suivantes :

- Mn = 29 800
- Mw = 84 700
- Tg = 45 °C

L'élution de la Nicergoline® effectuée dans les conditions de l'exemple 16 montre, sur la figure 2 {courbe (4){, une libération très faible pendant un temps de latence de 21 jours. Ceci est caractéristique de la dégradation à coeur des PLA-DL. Ensuite la Nicergoline® se libère beaucoup plus rapidement.

- EXEMPLE 18 :

Libération de Nicergoline® :

On opère comme dans l'exemple 16 mais en remplaçant le copolymère A par une composition élastomère silicone réticulable par réaction de polyaddition au platine et commercialisé par la Société RHONE-POULENC sous la dénomination commerciale RHODORSIL® RTV 141.

La réticulation de RTV 141 se fait à 120 °C pendant une heure et nul compactage n'est nécessaire pour obtenir un film de 1,5 mm d'épaisseur.

L'élution de Nicergoline® effectuée dans les conditions de l'exemple 16 montre sur la figure 2 {courbe (5)}, une libération quisi-constante de la Nicergoline® pendant au moins 70 jours, mais à un rythme lent.

La comparaison des exemples 16, 17 et 18 montre que dans les mêmes conditions, le copolymère A permet la libération totale du principe actif et à un rythme beaucoup plus rapide que ne le permet un élastomère silicone pur.

D'autre part, on évite le temps de latence lié à l'utilisation du PLA-DL.

- EXEMPLE 19 :

Libération de la Bovalbumine (BSA) :

On opère de façon identique à l'exemple 13, en remplaçant le Kétoprofène® par de la BSA broyée, de granulométrie moyenne égale à 35 µm. Le film obtenu après compactage à 70 °C est placé dans une enceinte thermostatée à 37 °C contenant 500 ml de solution tamponnée de composition suivante :

| | |
|---|---|
| - $H_2O$ | 1 000 g |
| - $Na_2HPO_4$, $12H_2O$ | 8,05 g |
| - $NaH_2PO_4$, $2H_2O$ | 2,03 g |
| - NaOH 4N | jusqu'à pH = 7,4 |

et sous faible agitation.

Le suivi de la cinétique de libération de la BSA se fait par dosage U.V. à 278 nm.

Les résultats obtenus et reportés sur la figure 3 {courbe (6)} montrent que la BSA est libérée à plus de 80 % en l'espace de quelques jours.

- EXEMPLE 20 :

Libération de la BSA :

On opère comme dans l'exemple 19 mais en remplaçant le copolymère A par un polylactique amorphe PLA-DL ayant les caractéristiques suivantes :
- Mn = 29 800
- Mw = 84 700
- Tg = 45 °C

L'élution de la BSA effectuée dans les conditions de l'exemple 19 montre, sur la figure 3 {courbe (7)} une libération rapide de celle-ci en 6 jours. Le profil de libération obtenu est à rapprocher de celui obtenu avec le copolymère A.

EXEMPLE 21 :

Libération de la BSA :

On opère comme dans l'exemple 19 mais en remplaçant le copolymère A par la composition élastomère silicone utilisée à l'exemple 18. La réticulation se fait à 120 °C pendant 1 heure et on ne compacte pas le film obtenu.

L'élution de la BSA effectuée dans les conditions de l'exemple 19 montre, sur la figure 3 {courbe (8)}, que celle-ci n'est pas libérée. En effet, la BSA qui est une macromolécule (poids moléculaire = 63 000) ne peut pas diffuser à travers l'élastomère silicone réticulé.

La comparaison des exemples 19, 20 et 21 montre que, contrairement à un élastomère silicone réticulé, le copolymère A permet la libération de macromolécules du type de la BSA et avec des profils de libération voisins de ceux obtenus avec une solution lactique commerciale.

- EXEMPLE 22 :

Utilisation d'une membrane en copolymère thermoplastique multiséquence polysiloxane/acide poly-L-lactique pour la libération controlée du Ketoprofène® :

1. - Préparation du copolymère : on utilise un oligomère poly-L-lactique $\alpha,\omega$-hydroxylé préparé dans les conditions suivantes :

Dans un réacteur de 1 litre équipé d'une agitation, d'un réfrigérant ascendant et balayé par un courant d'azote, on charge :
- 288 g de dimère-L-lactique cristallisé,
- 9 g d'éthylèneglycol distillé,
- 500 ml de toluène distillé,
- 0,5 g d'octoate stanneux.

Ce mélange est chauffé à léger reflux, sous agitation, pendant 5 heures.

Il est ensuite concentré sous pression réduite, vers 80-90 °C, dans un évaporateur rotatif.

Après dilution avec 150 ml de dichlorométhane, la solution est précipitée dans 2 litres d'eau déminéralisée, à 65 °C. On recueille, après filtration et séchage une nuit, sous pression réduite, à 45 °C : 297 g d'un solide blanc.

Caractérisation du produit :
- dosage des fonctions OH (totales) :

124,8 milliéquivalents/100 g,
- dosage des fonctions $CO_2H$ : 7,6 milliéquivalents/100 g.

On prépare ensuite le copolymère de la façon suivante :

Dans un réacteur tricol de 500 ml, en verre, équipé d'un agitateur, d'un réfrigérant ascendant et d'une ampoule de coulée, on charge :
- 130 g de l'oligomère poly-L-lactique précédemment décrit,
- 94,7 g d'un oligomère poly(diméthylsiloxane) $\alpha,\omega$-hydroxypropylé à 161 milliéquivalents OH/100 g,
- 25,62 g de diisocyanate-1,6 hexane,
- 230 ml d'ortho-dichlorobenzène.

Le mélange est agité et chauffé. Vers 110 °C, on ajoute 0,125 ml de STAVINOR 1200 SN (catalyseur). Ensuite, on poursuit le chauffage sous agitation, à 120 °C, pendant 6 heures. On élimine ensuite une partie du solvant à l'évaporateur rotatif à 120-130 °C, sous 2 mmHg. On dilue ensuite avec 250 ml de dichloro-1,2-éthane, puis on précipite dans 3 litres d'hexane. Après séchage à l'étuve, sous pression réduite, on obtient un solide blanc (233,6 g).

Le produit a été caractérisé par chromatographie par perméation de gel (GPC) :
- Mn = 12 250 g/mole,
- m$\omega$ = 37 990 g/mole (Etalonnage polystyrène).

a) - Conditions de pelliculage : on utilise un appareil de pelliculage en lit fluidisé UNI-GLATT® équipé d'un système WURSTER.

On prépare 350 g de kétoprofène® obtenus de la manière suivante :

Matières premières :
- Avicel PH 101, FMC (E.U.),
- Courlose, Courtaulds Acetate Ltd (G.B.).

Kétoprofrène® B.P., RHONE-POULENC Ltd (G.B.).

Les granulés ont été préparés selon un procédé d'extrusion-sphéronisation. 3 kg de kétoprofène® ont été mélangés avec 965,6 g d'AVICEL PH 101 dans un mélangeur planétaire HOBART à la vitesse de 1. Ce mélange de poudre a été granulé avec 2 363 g d'une solution de Courlose à 1,5 % (p/p) pendant 2 minutes. La masse humide a été extrudée dans un appareil Fuji Paudal AXDCS-700, commercialisé par RUSSEL FINEX. Cet appareil était muni d'une grille percée de trous de 1 mm de diamètre et de 1 mm d'épaisseur. Les corps extrudés cylindriques ont été introduits dans une machine à rouler des granulés sphériques (Spheronizer JB Caleva) pendant 3 minutes et à une vitesse de 500 tours par minute. Ils ont ensuite été tamisés dans un appareil du type FRITSCH SIEVE ANALYSER pour que leur taille soit comprise entre 800 et 1 400 µm.

L'appareil est chargé avec 350 g de mini-granulés de kétoprofène®.

On pulvérise une solution préparée avec :
- 39 g de copolymère de polysiloxane et d'acide poly-L-lactique,
- 707 ml de dichloro-1,2-éthane PROLABO RECTAPUR®

Les conditions de pulvérisation sont les suivantes :
- débit d'air de fluidisation : 72 $m^3$/h,
- température de l'air de fluidisation : 40 °C,
- pression de l'air de pulvérisation : 1,5 bar,
- température de l'air de pulvérisation : ambiante,
- débit de solution d'enrobant : 3 ml/mn.

On prélève dans l'appareil des échantillons de granulés enrobés, après les d'durées de pulvérisation correspondant respectivement aux taux d'enrobant calculés de 3,6 à 10 % (essais A, B, C).

b) - Cinétique de libération du kétoprofène® en milieu tamponné (pH = 6.6). à 37 °C :

Ces trois échantillons ont été soumis au test de libération suivant :

Description du test : dans un réacteur en verre de 1 litre muni d'un agitateur et chauffé par un bain d'eau thermostaté à 37 °C, on charge 6 g de granulés et 1 litre de solution à pH = 6,6.

La solution tampon utilisée a été préparée de la manière suivante :
- dissolution de 68 g de dihydrogénophosphate de potassium ($KH_2PO_4$) dans 10 litres d'eau déminéralisée,
- ajustement du pH à 6,6 par coulée d'une solution de soude N.

Les courbes cinétiques ont été tracées à partir des dosages de kétoprofène® réalisés à 260 nm, sur des prélèvements de la phase liquide, avec un spectrophotomètre UV/visible PHILIPS de type PYE UNICAM PU 8600.

La vitesse d'agitation de la suspension de granulés était de 300 rpm.

Les résultats de ces tests sont présentés dans le tableau 2 ci-après.

| ENROBAGE DE KETOPROFENE® | | | | |
|---|---|---|---|---|
| Cinétique à pH 6,6 et 37 °C<br>% KETOPROFENE® libéré | | | | |
| Granulés | NON ENROBES | A | B | C |
| Taux d'enrobant | 0 | 1,5 % | 4,6 % | 9,2 % |
| 30 minutes | 42,0 % | | | |
| 1 heure | 55,0 % | 7,5 % | 3,9 % | 2,5 % |
| 2 heures | 70,5 % | 14,2 % | 8,3 % | 4,8 % |
| 3 heures | 80,0 % | 21,1 % | 12,4 % | 7,1 % |
| 4 heures | 85,0 % | 28,1 % | 15,6 % | 10,0 % |
| 5 heures | | 32,6 % | 21,4 % | 11,9 % |
| 6 heures | | 37,6 % | 24,3 % | 14,1 % |
| 7 heures | 96,0 % | 43,2 % | 28,2 % | 16,8 % |
| 8 heures | | 47,6 % | 32,6 % | 18,8 % |
| 16 heures | | 73,7 % | 54?4 % | 38,5 % |
| 18 heures | | 79,3 % | 58,9 % | 43,5 % |
| 20 heures | | 82,1 % | 62,8 % | 46,7 % |
| 22 heures | | 87,6 % | 67,5 % | 51,0 % |
| 24 heures | | 89,7 % | 70,7 % | 52,5 % |
| 26 heures | | 92,8 % | 77,3 % | 55,6 % |
| 28 heures | | 94,0 % | 78,6 % | 58,8 % |
| 30 heures | | 94,0 % | 81,2 % | 62,0 % |
| 32 heures | | 94,0 % | 84,2 % | 63,9 % |
| 40 heures | | 98,5 % | 89,9 % | 75,8 % |
| 42 heures | | | 90,5 % | |
| 44 heures | | | 92,3 % | |
| 46 heures | | | 94,1 % | |
| 48 heures | | | 94,6 % | |
| 50 heures | | | 93,7 % | |
| 52 heures | | | 96,7 % | |
| 54 heures | | | | |

## Revendications

1. - Copolymère à blocs diorganopolysiloxane-polyester présentant de 1 à 99 % en poids de segments diorganopolysiloxane de formule moyenne :

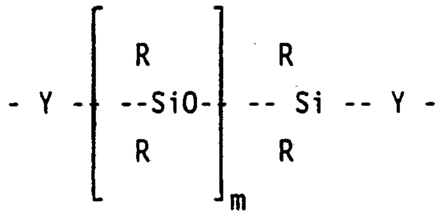 (1)

et de 99 à 1 % en poids de segment polyester de formule moyenne :

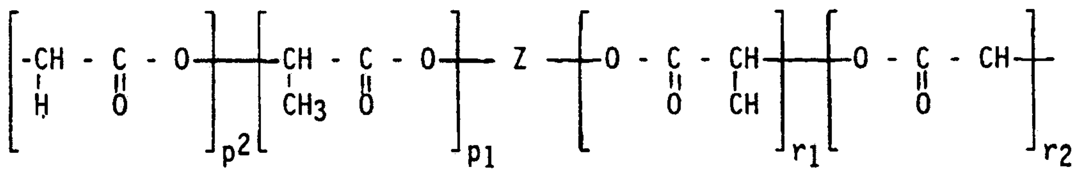 (2)

dans lesquelles :
- les radicaux R, identiques ou différents, sont des radicaux organiques monovalents,
- Y représente un radical organique divalent relié à l'atome de silicium par une liaision SiC,
- Z est un radical hydrocarboné divalent de formule :
- $CH_2$ - W - $CH_2$ -,
dans laquelle W est choisi parmi un radical hydrocarboné divalent linéaire, ramifié ou cyclique ayant de 1 à 8 atomes de carbone et une liaison covalente,
- m est un nombre entier compris entre 1 et 500, - $p_1$, $p_2$, $r_1$ et $r_2$ sont des nombres entiers compris entre 0 et 10 000 inclus avec :
$r_1 + r_2 + p_1 + p_2$ supérieur à 1 et inférieur à 10 000,
- les segments (1) et (2) étant reliés entre eux par un pont de formule :

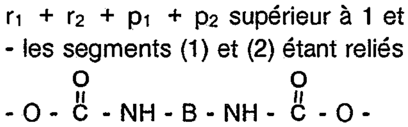 (3)

où B est un radical organique ou organosilicié divalent pouvant éventuellement comporter un segment diorganopolysiloxane de formule moyenne (1 bis).

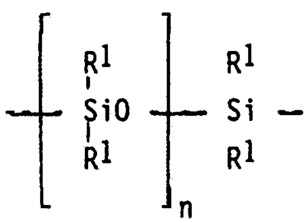 (1 bis)

dans laquelle $R^1$ a la même signification que R et n a la même signification que m, R et $R^1$, me et n pouvant avoir des significations identiques ou différentes.

2. - Copolymère selon la revendication 1, caractérisé en ce que les radicaux R sont choisis parmi les radicaux méthyle, W est une liaison covalente, et en ce qu'il comporte de 10 à 90 % en poids de segments diorganopolysiloxane et de 90 à 10 % en poids de segments polyesters, et en ce que $r_1 + r_2 + p_1 + p_2$ est compris entre 10 et 200 inclus.

3. - Copolymère selon la revendication 1 ou 2, caractérisé en ce qu'il ne comporte pas de segment de formule (1) quand B comporte un segment de formule (1 bis).

4. - Procédé de préparation d'un copolymère à blocs tel que défini à la revendication 1 ou 2, caractérisé en ce qu'on fait réagir :

a) - un oligomère diorganopolysiloxane de départ répondant à la formule générale :

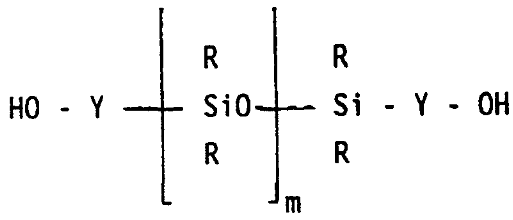 (4)

dans laquelle les symboles Y, identiques ou différents, représentent un radical organique divalent relié à l'atome de silicium par une liaison SiC et R et m a la même signification que ci-dessus.

b) - un oligomère polyester de départ répondant à la formule générale moyenne :

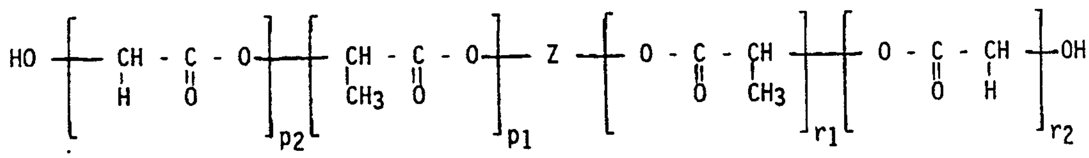 (5)

dans laquelle Z, $p_1$, $p_2$, $r_1$ et $r_2$ ont la même signification qu'à la formule (2) ci-dessus.

c) - un composé diisocyanato de formule :

O = C = N - B - N = C = O (6)

dans laquelle B symbolise un radical organique ou organosilicié divalent pouvant comporter un segment de formule (1 bis) ci-dessus.

5. - Procédé de préparation d'un copolymère àblocs alternés tel que défini à la revendication 3, caractérisé en ce que :

- au cours d'une première étape on fait réagir l'oligomère polyester b) de formule (5) sur un excès molaire d'un composé oléfinique porteur d'un groupement isocyanate en particulier décrit dans le brevet européen EP-A-77 744, de préférence répondant à la formule générale :

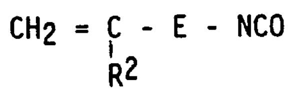 (7)

dans laquelle $R^2$ est un radical alkyle en $C_1$-$C_6$, E est un radical hydrocarboné exempt d'insaturation oléfinique, ayant de 1 à 20 atomes de carbone, et pouvant comporter au moins un hétéroatome choisi parmi O et Si, de préférence au sein d'un solvant organique en présence d'un catalyseur à l'étain tel que le dilaurate de dibutylétain.

- au cours d'une deuxième étape on fait réagir l'oligomère polyester (bis-$\alpha,\omega$ vinyle) obtenu au cours de la première étape sur un diorganopolysiloxane de formule moyenne :

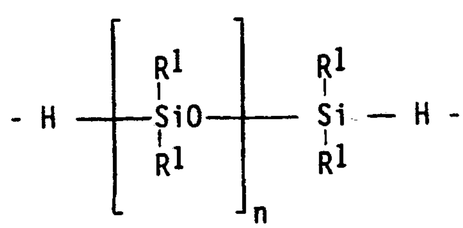 (8)

dans laquelle $R^1$ et n ont la signification donnée à la formule (1 bis) ci-dessus, en présence d'une quantité catalytiquement efficace d'un catalyseur d'hydrosilylation, de préférence un catalyseur au platine suivant un rapport molaire SiH compris entre 0,9 et 1,5.

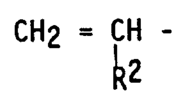

6. - Procédé selon la revendication 5, caractérisé en ce que le produit de formule (7) est l'allylisocyana-

te de formule :

$CH_2 = CH - CH_2 - NCO$

7. - Copolymère à bloc susceptible d'être obtenu par la mise en oeuvre d'un procédé tel que défini à l'une quelconque des revendications 4 à 6.

8. - Objets dégradables par hydrolyse, caractérisés en ce qu'ils sont constitués au moins en partie par un copolymère tel que défini à l'une quelconque des revendications 1 à 3 et 7.

9. - Objets dégradables par hydrolyse selon la revendication 8, comportant en outre un principe actif.

10. - Objets selon la revendication 9, caractérisés en ce que le principe actif est encapsulé par le copolymère.

11. - Objets selon la revendication 9, caractérisés en ce que le principe actif est dispersé dans une matrice de copolymère.

12. - Objets selon l'une des revendications 9 à 11, caractérisés en ce que le principe actif est choisi parmi un principe actif médicamenteux, un produit phytosanitaire, un embryon végétal, une semence végétale, un catalyseur et un produit cosmétique.

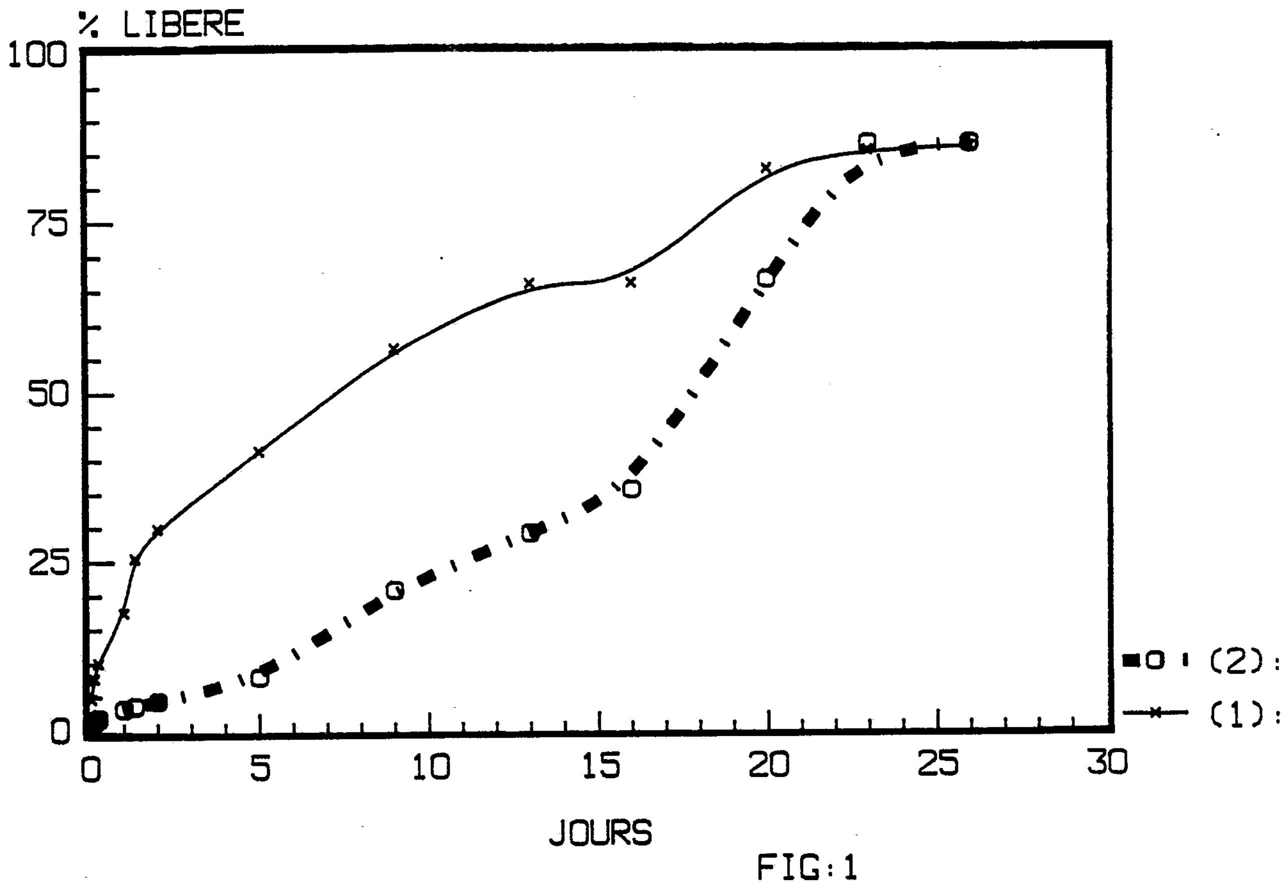


FIG:1

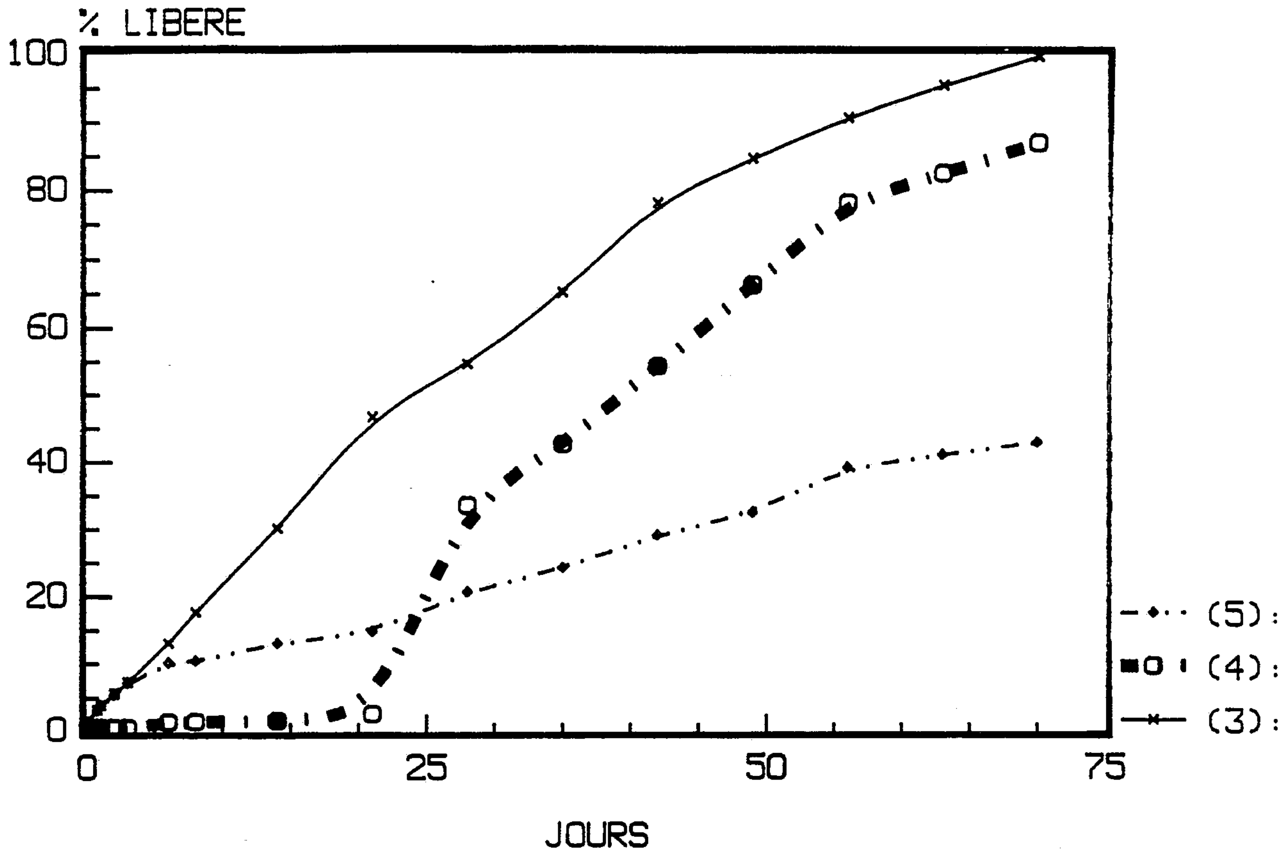


FIG:2

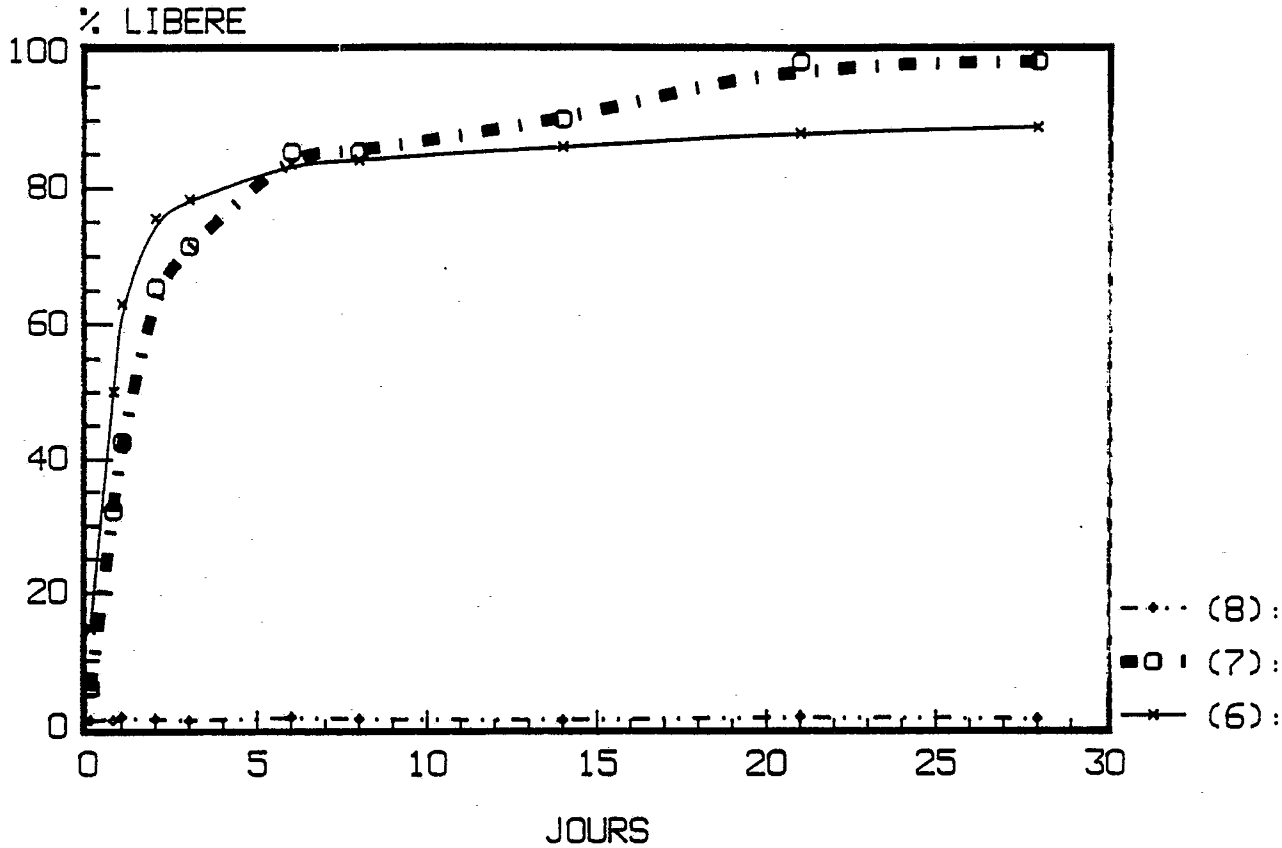


FIG:3

Office européen des brevets

# RAPPORT DE RECHERCHE EUROPEENNE

Numero de la demande

EP 90 42 0107

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EP-A-0 299 730 (MITSUI TOATSU)<br>* Revendication 1 * | 1 | C 08 G 77/445 |
| A | EP-A-0 165 849 (RHONE-POULENC)<br>* Revendication 1 * | 1 | |
| A | GB-A- 993 493 (ICI)<br>* Revendication 1 * | 1 | |
| | | | DOMAINES TECHNIQUES RECHERCHES (Int. Cl.5) |
| | | | C 08 G |

Le présent rapport a été établi pour toutes les revendications

| Lieu de la recherche | Date d'achèvement de la recherche | Examinateur |
|---|---|---|
| LA HAYE | 06-07-1990 | DEPIJPER R.D.C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)